# EUROPEAN PATENT APPLICATION

(11) **EP 4 056 271 A1**
(43) Date of publication of application: **14.09.2022**
(21) Application number: 21161887.1
(22) Date of filing: 10.03.2021
(51) Int. Cl.: B01L 3/00, G01N 33/53, G01N 33/574

(54) **QUANTIFICATION OF CELL MIGRATION AND METASTATIC POTENTIAL OF TUMOR CELLS**

(71) Applicant: Cline Scientific AB, 413 53 Mölndal (SE)
(72) Inventor: Sundh, Patrik, 423 41 TORSLANDA (SE); Evenbratt, Hanne, 443 95 STENKULLEN (SE); Holmquist, Niklas, 434 91 KUNGSBACKA (SE); Andäng, Michael, 146 54 TULLINGE (SE); Vincent, Theresa, 114 42 STOCKHOLM (SE); Szeszula, Petra, 134 62 INGARÖ (SE); Mobini, Reza, 413 08 GÖTEBORG (SE)
(74) Representative: Ström & Gulliksson AB

(57) **Abstract**

The present invention relates to a gradient-on-a-chip device for quantification of cell migration and metastatic potential of tumor cells. The device comprises a chip having a chip surface, and a nano gradient layer of nanoparticles provided on the chip surface. The nano gradient layer having a gradient direction along an axis of an X-Y plane of the chip surface. The device further comprises a biomolecule conjugated to the nanoparticles and a linker conjugated to the nanoparticles. The linker being adapted to link together the biomolecule to the nanoparticles. The chip surface has at least one guiding structure arranged to guide the tumor cells in the gradient direction, the guiding structure extending in the gradient direction and delineating a migration corridor comprising the nano gradient layer.

## Description

### FIELD OF THE INVENTION

The present disclosure relates to the field of live cell culture, the quantification of cell migration, and the metastatic potential of tumor cells.

### BACKGROUND

Cancer is the second largest worldwide cause of death and poses the highest clinical, social, and economic burden among all human diseases. The majority of cancer deaths are caused by metastases, where the tumor cells have detached from the primary tumor and spread to distant organs.

Existing solutions to predict the outcome of breast tumors and the likelihood of metastasis have emerged in recent years. These include classical pathology (e.g., histopathology and MRI/PET scan), and biomarkers (e.g., the presence of receptors for estrogen (ER), progesterone (PR), and human epidermal growth factor receptor 2 (HER2)). However, classical pathology only detects metastases in advanced stages and while biomarkers are used to classify breast cancer and provide information on whether hormonal treatment would be advantageous and prediction of recurrence, they do not directly determine metastatic potential. Significant efforts have been made to develop high-throughput molecular technologies to identify molecular markers such as gene signatures to guide decision-making processes (e.g. PAM50, MammaPrint). New biomarkers aim at discovering metastasis but are limited by detecting single or a small set of genes or proteins, which provides only indirect indication of potential metastasis in specific subclasses of breast cancer. Moreover, interpretation and clinical translation of results from multiple genomic and proteomic analysis variables remain a significant challenge. Genetic testing can be expensive, time-consuming, and limited due to the biasing presence of normal cells and intratumoral heterogeneity. These methods have the drawback of only providing a snapshot of different factors and do not quantify the real-time migration and behavior of living cells.

Ideally, reliable diagnostics could help to distinguish invasive tumor cells and, subsequently, to define appropriate treatment strategies at an early stage. However, a method that can definitively and efficiently determine whether a primary tumor will metastasize does not exist today and the ability to tailor the patient's therapy is therefore limited. Further, the ability to predict and understand the mechanisms of metastasis would be of great value in the search for metastasis limiting pharmaceutical therapies. Chemotaxis studies have been crucial for the study of cell migration and tumor metastasis. One way to investigate and predict intratumoral cells' metastatic behavior is with the use of biomolecular gradients. Biomolecular gradients are established by a difference in the concentration of molecules in a biological system and are important for communication, adhesion, and morphology of cells. Different methods to provide chemical gradients have been established over the years. However, they are limited in their ability to produce precise and consistent enough controlled gradients for such studies.

Traditional methods of producing biomolecule gradients (gradient chambers, micropipette-generated, biological hydrogel) have been useful instrumentals in scientific research, however, they are limited in their ability to produce precise and quantitative results necessary for a diagnostic product. Biomolecule gradients constructed in free liquid solutions can behave inconsistently and diffuse over time and are highly susceptible to small vibrations, thermal imbalances, or evaporation. For biological hydrogel based gradients, the use of cell-based biomolecule sources such as explants or transfected cell lines to form gradients leads to high variability in resultant gradients (Keenan TM, Folch A. Biomolecular gradients in cell culture systems. Lab Chip. 2008 Jan;8(1):34-57. doi: 10.1039/b711887b. Epub 2007 Dec 6. PMID: 18094760; PMCID: PMC3848882). These limitations mean that cells are not exposed to a constant, precise user-defined gradient and results are often not quantitative and not of adequate precision, both of which necessary for a diagnostic product.

Moreover, existing cell migration assays do not replicate the real-life tumor conditions and are therefore not an accurate representation of tumor cells in vivo. Further, they are not able to combine multiple factors and biomolecule gradients, a key competent in order to replicate *in vivo* cellular conditions.

In recent years microfluidic technology has been developed for chemotaxis and cell migration studies and a proposed way to overcome above noted limitations. An example of this is US 2018 0 280 976, which relates to gradient-on-a-chip which discloses a cell culture device and its applications of creating gradient of chemicals or gradient of cells to mimic the in vivo physiological conditions of homeostasis.

Some work has been done to apply the technology to determine metastatic potential of cells derived from cancer patients. Examples include US 2016 0 158 751 and US 2020 0 249 232 which discloses the use of an integrated microfluidic chip for analysis of cell motility and prediction and prognosis of patient survival, with or without a chemical gradient.

However, these newer methods also have drawbacks. Disadvantages of microfluidics-based method include their cost, complexity, and that the forces and pressures present could be disturbing or damaging to cells. These are still subject to inconsistency in gradient and conditions for the cells due to the fluidic nature of the apparatuses.

Surface bound nano-gradients overcome many of the issues that the field has faced in creating a precise and controllable continuous gradient. One example is the device disclosed in WO 2012/025576.

Nevertheless, there exists a need for a more effective device and method for measuring tumor cell migration and determine the metastatic potential of tumors cells.

### SUMMARY

An object of the disclosure is to provide a method and device that can be used to measure migration capacity, i.e. the movement of a cell, and metastatic potential of tumor cells in vitro.

According to a first aspect of the disclosure, these and other objects are achieved, in full or at least in part, by a gradient-on-a-chip device for quantification of cell migration and metastatic potential of tumor cells. The device comprises:
- a chip having a chip surface;
- a nano gradient layer of nanoparticles provided on the chip surface, the nano gradient layer having a gradient direction along an axis of an X-Y plane of the chip surface;
- a biomolecule conjugated to the nanoparticles; and
- a linker conjugated to the nanoparticles, the linker being adapted to link together the biomolecule to the nanoparticles;
characterized in that
the chip surface having at least one guiding structure arranged to guide the tumor cells in the gradient direction, the guiding structure extending in the gradient direction and delineating a migration corridor comprising the nano gradient layer.

As explained in the background section, nano gradients constitute a useful tool to induce cell migration. The nano gradient layer according to the invention may be described as a dispersion of nanoparticles onto a chip surface in a gradient pattern, where density of particles increases along one axis of the chip surface. The nano gradient layer may for example consist of nano-patterns of gold nanoparticles, onto which biomolecules of interest are attached. It is created to mimic tissue function at the molecular level, e.g. intra-tumoral spaces. The gradient layer may be utilized to influence and direct cell migration along the gradient direction of the nano gradient layer. It may also be utilized to screen for sensitive reactions and/or interactions between cells and biomolecules, thus determining the optimal composition of biomolecules responsible for a certain cellular response. The region on the gradient responsible for a reaction may then be recreated in a nano layer. A method for producing the nano gradient layer is described in WO 2012/025576.

However, the measurement of cell migration in a nano gradient layer can be problematic due to interactions between cells. Such cell-cell interactions occur when adjacent cells get in contact, and often causes the cells to change its migration path, possibly in a direction opposite the gradient direction of the nano gradient. The cell-cell interactions thus disturb the migration behavior of the cells. Furthermore, cell-cell interactions may complicate screening and analyzation of the interactions between cells and biomolecules.

The more cells present in a specified area, the greater risk that disturbing cell-cell interactions will occur when the cells starts to migrate. Measurements has shown that severe cell-cell interactions may disturb cell migration to such an extent that it is difficult, if not even possible, to retain accurate analyzation results of cells migration behavior. Cell-cell interaction may also cause disturbance when an optimal composition between cells and biomolecules is to be determined.

The inventors have solved this problem by a guiding structure arranged in the gradient direction. The guiding structure extending in the gradient direction is meant to be understood as the guiding structure extending substantially parallel with the gradient direction.

The function of the guiding structure is to delineate a migration corridor for the cells. Hereby, the migration corridor is delimited by the guiding structure. A migration corridor may be defined as the space between the guiding structure and the outer edge of the chip surface. A device having only one single guiding structure may thus contain two migration corridors, the two of them located on each side of the guiding structure. Alternatively, the space located between two adjacent and parallel guiding structures may define a migration corridor.

The migration corridor is adapted to guide the cells in the gradient direction along the nano gradient layer and to prevent cells from interacting with each other. Hereby, the area for each cell to migrate in is delimited. The device thus has the advantage that it eliminates disturbance from cell-cell interactions.

Moreover, the migratory behavior of tumor cells within a migration corridor may be more correctly and reliantly measured and recorded than without migration corridors, which enhances the capability of predicting cells ability to spread and metastasize. A further advantage is that the time for measurement may be shortened when cell-cell interactions are avoided.

According to one embodiment, the nanogradient layer is a continuous nanogradient layer. This means the concentration of nanoparticles increase continously in the gradient direction.

According to at least one example embodiment, the guiding structure is a ridge extending out of the chip surface or a recession pointing into the chip surface. Further non-limiting examples of guiding structures are cuts, scores, notches, or incisions in the chip surface. These examples illustrate guiding structures that are contained in the chip surface. Alternatively, the chip surface may be modified with excess material to create a guiding structure in the form of a ridge or a wall. The guiding structure may contain the same material as the chip and/or a different material.

According to at least one example embodiment, the guiding structure extends continuously along the chip surface.

This is to be interpret as the guiding structure extending continuously along the migration corridor. Hereby, the delineation of the migration corridor is continuous along the gradient direction of the chip surface.

In an embodiment, the guiding structure extends continuously along a portion of the chip surface. Alternatively, the guiding structure may extend discontinuously along the corridor. There may for example be breaks in the guiding structure along the gradient direction.

According to at least one example embodiment, the device further comprises a chip surface area void of nano gradient layer, and wherein the guiding structure is the boundary line between the migration corridor and the chip surface area void of nano gradient layer.

This is to be interpret as the migration corridor being delimited by a surface area void of nano gradient layer. An area void of nano gradient layer also means an area void of biomolecules. Such an area is not pleasant for the cells to migrate into, or uphold themselves in, as they prefer to interact with the biomolecules and thus follow the gradient direction.

According to at least one example embodiment, the migration corridor has a substantially constant width along its extension direction.

The migration corridor has thus equal width in both end points. By having a constant width along the extension direction, the cells are induced to travel linearly in the gradient direction.

According to at least one example embodiment, the migration corridor has a width in the range from 20-500 µm, and preferably from 50-200 µm.

According to an embodiment, the migration corridor has a width in the range from 30-120 µm, more preferably 40-80 µm, and most preferably 50-60 µm.

Hereby, the width of the corridor matches a cell size of about 50 µm. By having a corridor width matching the size of the cell, linear cell migration may be induced. A corridor having the width of 50 µm is preferably used for the measurement of a single cell or a low number of cells, e.g. that have been loaded at different loading areas along the gradient direction. A wide corridor has the advantage that a plurality of cells may be present in the same corridor.

According to at least one example embodiment, the gradient-on-a-chip device comprises two or more migration corridors. Two or more migration corridors allow simultaneous measurement of the migration behavior of a plurality of cells. This is beneficial since it saves time for analyses. The more migration corridors that are present, the more cells may be measured simultaneously.

According to at least one example embodiment, the gradient-on-a-chip device further comprises at least 50 migration corridors, at least 100 migration corridors, or at least 150 migration corridors. The number of migration corridors may be correlated to the size of the chip. A large chip has the benefit that more corridors may be present than in a small chip.

According to an embodiment, the device comprises two or more guiding structures.

According to an embodiment, the device comprises at least 25 guiding structures, preferably at least 50 guiding structures, and most preferably at least 100 guiding structures.

According to at least one example embodiment, the two or more migration corridors are of equal width.

This may be achieved by providing a single guiding structure provided in the center of the chip surface, the guiding structure delineating two separate migration corridors provided on each side of the guiding structure.

An advantage of having two or more migration corridors of equal width is the results of different cells can be compared. A further advantage may be that the manufacturing could be easier och cheaper.

Alternatively, the device comprises two or more migration corridors of different widths.

According to an embodiment, the biomolecule is an epidermal growth factor (EGF). Other non-limiting examples of biomolecules are Semaphorin-3E (Sema3E), Semaphorin-4D (Sema4D), Semaphorin-5a (Sema5a), C-C motif chemokine 27 (CCL27), C-C motif chemokine 38 (CCL38), C-C motif chemokine 48 (CCL48), C-C motif chemokine 58 (CCL58), C-C motif chemokine 12 (CCL12), C-C motif chemokine 199 (CCL199), C-C motif chemokine 21 (CCL21), C-C motif chemokine 22 (CCL22), C-C motif chemokine 25 (CCL25), C-X-C motif chemokine 5 (CXCL5), C-X-C motif chemokine 8 (CXCL8) (IL-8), C-X-C motif chemokine 9 (CXCL9), C-X-C motif chemokine 10 (CXCL10), C-X-C motif chemokine 12 (CXCL12), C-X-C motif chemokine 13 (CXCL13), C-X-C motif chemokine 14 (CXCL14), Interleukin-11 (IL-11), Fibroblast growth factor (FGF), Platelet-derived growth factor (PDGF), Placenta growth factor (PIGF), Hepatocyte growth factor (HGF), HB-EGF (Heparin-binding, EGF-like), Slit homolog 2 protein (Slit2), Vascular endothelial growth factor a (Vegf-a), Vascular endothelial growth factor b (Vegf-b), Vascular endothelial growth factor c (Vegf-c), Ephrin type-A receptor 2 (EphA2) (Eph - receptor), and Ephrin type-B receptor 4 (EphB4). In regard to mimicking the intratumoral environment, these biomolecules may be combined with ECM fibers such as fibronectin or laminin.

In one embodiment, the chip surface, or parts of the chip surface, is coated by a coating agent between the nanoparticles. An example of coating agent is fibronectin.

According to at least one example embodiment, the linker comprises the linker complex biotin/streptavidin.

In one embodiment, the nanoparticles are gold particles coated with thiolated streptavidin, and the biomolecules are biotinylated and attached to the nanoparticles through biotin/streptavidin interaction. However, any such known principle for facilitating binding and interaction between nanoparticles and a biomolecule may be used, since the biotin/streptavidin interaction is only an interaction according to one embodiment. For instance, such interaction may be formed using an ion-exchange/interaction, hydrophobic interactions and dative binding, covalent binding of thiols, carbo di-imide chemistry, bi-functional linkers (such as EDC/NHS chemistry) or click chemistry.

According to at least one example embodiment, device further comprises two or more migration corridors provided on top of each other. This may be achieved by two nano gradient layers provided on top of each other.

According to a second aspect of the disclosure, a method for quantification of cell migration and metastatic potential of tumor cells is provided. The method comprises the steps of:
- applying at least one tumor cell to the gradient-on-a-chip device in claim 1;
- measuring and recording the cell migration of the tumor cell for a repeated time period of 2-48 hours, preferably 3-24 hours, and most preferably 3-12 hours.

Measuring and recording means that the migration behavior of the cells are documented. Non-limiting examples of features that may be measured and recorded are migration velocity, migration distance, migration motility and migration direction.

The method according to the inventive concept has the advantage that it may generate results within hours, therefore saving time and costs associated with diagnosis. The added information about the nature of the tumor behavior and risk allows for better targeted and earlier treatment than otherwise possible.

With this method it may be possible to screen and follow the migration behavior of tumor cells while elucidating the disturbance from cell-cell interactions. The method makes it possible to better analyze the interactions between the cells and the biomolecules, e.g. to analyze the impact the biomolecule provides to the cell migration. Furthermore, the method makes it possible to evaluate different biomolecules and evaluate pros and cons for different types of molecules in order to find out best possible alternative to predict metastasis potential of tumor cells.

The tumor cell may be applied within a migration corridor. The tumor cell may for example be applied in the beginning of the migration corridor. Alternatively, the cell may be applied outside a migration corridor.

According to an embodiment, the tumor cell is applied in an end of the migration corridor having the lowest nanoparticle concentration. Hereby, the cell is induced to migrate from an area with lower concentration of biomolecules to an area of higher concentration of biomolecules.

Alternatively, the cell is applied in an area of intermediate nanoparticle concentration, i.e., an area located in between the highest concentration of nano particles and the lowest concentration of nanoparticles.

According to at least one example embodiment, the measuring and recording is performed by imaging and/or isotope analysis.

According to at least one example embodiment, the tumor cells are breast cancer cells, melanoma cancer cells, prostate cancer cells, colorectal cancer cells, or lung cancer cells.

According to a third aspect of the disclosure, the use of the gradient-on-a-chip device according to the first aspect for quantification of cell migration and metastatic potential of tumor cells is provided.

According to at least one example embodiment, the tumor cells are breast cancer cells, melanoma cancer cells, prostate cancer cells, colorectal cancer cells, or lung cancer cells . Non-limiting examples of features that may be measured and recorded are migration velocity, migration distance, migration motility, and migration direction.

The present invention provides a gradient with multiple biomolecules and features that mimic conditions in vivo. This unlocks the ability to study tumor cells in real-time, with tumor microenvironment features and use this information to determine the metastatic potential of cell populations.

The device and the method can be used in a laboratory as a part of a diagnostic product on live patient tumor cells derived from a biopsy test at multiple points during a patient's cancer diagnostic, treatment, and monitoring.

### Definitions

Gradient is in this context meant concentration or density gradient, defined as the difference in the concentration or density of a substance between two areas.

A nano gradient layer is a surface covered with nanoparticles of which the distance between adjacent nanoparticles increase, or decrease, in a given direction from a given reference point. A nano gradient layer may also be interpreted as a dispersion of nanoparticles onto a surface in a gradient pattern.

The term nanoparticle is in this context meant particles of size between 1 and 100 nanometers (nm) in diameter.

The term nano gradient layer is surface covered with nanoparticles of which the distance between adjacent nanoparticles increase, or decrease, in a given direction from a given reference point.

The term continuous nano gradient layer is surface covered with nanoparticles of which the distance between adjacent nanoparticles increase, or decrease, continuously in a given direction from a given reference point.

The term gradient direction is in this context interpreted as the direction in which the distance between adjacent nanoparticles decrease or increase from a given reference point.

Generally, all terms used in the claims are to be interpreted according to their ordinary meaning in the technical field, unless explicitly defined otherwise herein. All references to "a/an/the [element, device, component, means, step, etc.]" are to be interpreted openly as referring to at least one instance of the element, device, component, means, step, etc., unless explicitly stated otherwise. The steps of any method disclosed herein do not have to be performed in the exact order disclosed, unless explicitly stated.

### BRIEF DESCRIPTION OF THE DRAWINGS

The disclosure will be described in more detail with reference to the appended schematic drawings, which show an example of a presently preferred embodiment of the disclosure.
Fig. 1 illustrates a gradient-on-chip device according to at least one example embodiment of the inventive concept;
Fig. 2 illustrates a gradient-on-chip device according to at least one example embodiment of the inventive concept;
Fig. 3 illustrates in perspective view the gradient-on-ship device in Fig. 2;
Fig. 4 illustrates the definition of motility, migration, Y-distance, and migration directness of a cell;
Fig. 5 illustrates cells migration directness;
Fig. 6 illustrates cells migration directness.

### DETAILED DESCRIPTION

The present disclosure will now be described more fully hereinafter with reference to the accompanying drawings, in which currently preferred embodiments of the disclosure are shown. The present disclosure may, however, be embodied in many different forms and should not be construed as limited to the embodiments set forth herein; rather, these embodiments are provided for thoroughness and completeness, and to fully convey the scope of the disclosure to the skilled addressee. Like reference characters refer to like elements throughout.

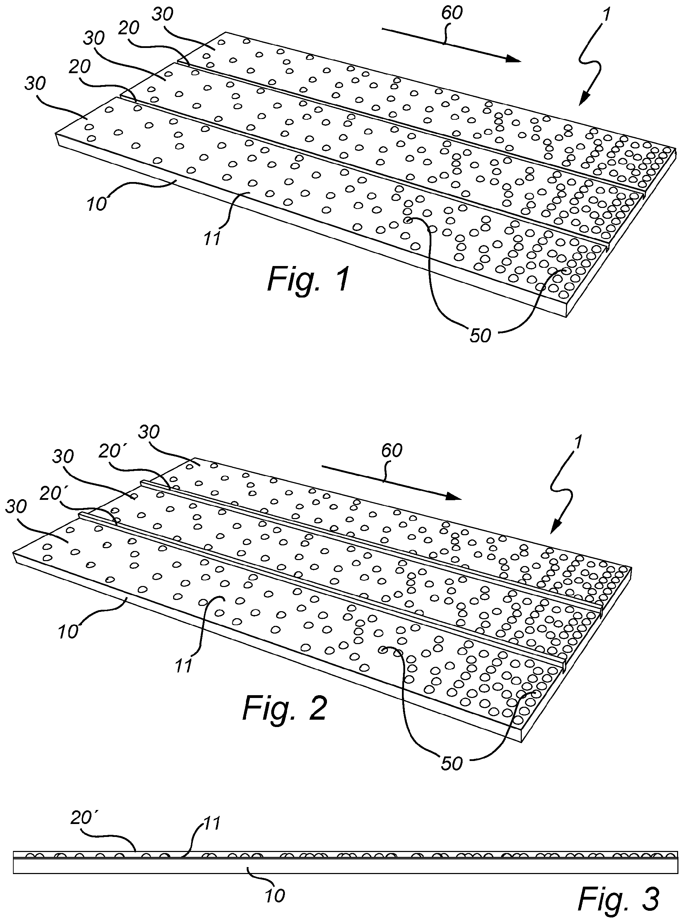

Fig. 1-3 illustrate a gradient-on-chip device 1 according to at least one example embodiment of the inventive concept. The device 1 comprises a chip 10 of which upper part constitutes a chip surface 11. The function of the chip 10 is to support a nano gradient layer applied thereto. The chip 10 may consist of metal, polymer, or ceramics, such as glass. The chip 10 may for example be a slide of glass or a slide of silicon.

The nano gradient layer extends over the entire chip surface 11 and comprises nanoparticles 50 provided in a gradient pattern. The nanoparticles 50 are for example gold nanoparticles 50. An advantage with gold particles is that gold is inert and easy to link biomolecules to.

The nanoparticles 50 have a biomolecule applied to them. The biomolecule is attached to the nanoparticles 50 via a linker conjugated to the nanoparticles 50. The function of the linker is to enhance the adherence of the biomolecule to the nanoparticles 50, and is preferably located such that the cells easily can attract to them, such that the cells are affected by the linker. The linker is for example biotin.

The nano gradient layer has a gradient direction in an axis of the X-Y plane of the chip surface 11, for example in the X-axis. The chip surface 11 which the nano gradient is provided on, comprises at least one guiding structure 20 arranged in the gradient direction. The device 1 in Fig. 1 and Fig. 2, respectively, has two guiding structures 20, 20'. The guiding structures 20 in Fig. 1 are recessions pointing into the chip 10 whereas the guiding structures 20' in Fig. 2 are ridges extending out of the chip surface 11. These guiding structures 20, 20' divide the chip surface 11 and the nano gradient layer in three separate migration corridors 30. When the tumor cells are applied in a migration corridor 30 it is, thanks to the guiding structure 20, 20', prevented from reaching a tumor cell applied in a different migration corridor 30. This guiding structure 20, 20' thus prevents cell-cell interactions.

In order measure cell migration, metastatic cells are provided within a migration corridor 30 of the device 1. The device 1 in Fig. 2 and Fig. 3 allows for three different cells to migrate in the gradient direction without disturbing each other if each one of them is are placed in a separate migration corridor 30. It should be noted that the device 1 allows for measurement of more than one cell per migration corridor 30. In an alternative embodiment, the device 1 may comprise more than 100 migration corridors 30.

The cells are preferably repeatedly measured and recorded mapped in order to quantify migration, e.g. rate, distance, direction and/or motility. Mapping may be either by imaging and/or by isotope analysis.

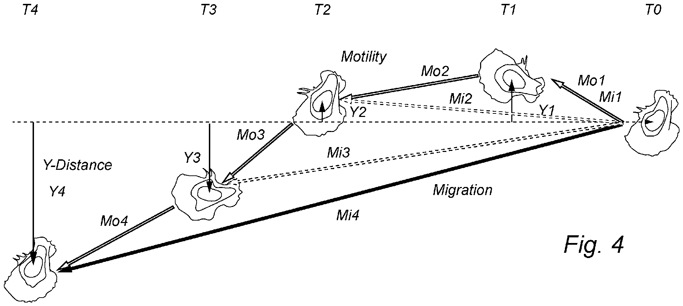

Fig. 4 illustrates different factors (and their relationship) that can be measures according to the inventive concept. The Y-Distance (µm) is defined by Y4 and is the change in the Y position of the cell. X position and Y position are expressed as coordinates on the XY plane measured in pixels. The motility (µm) is the sum of distances Mo1 + Mo2 + Mo3 + Mo4. Motility speed is the change in position over time, measured as micrometer per hour. Migration (µm) is defined as Mi4 and relates to the movement of a cell in a direction along the axis of the gradient. Migration directness is the ratio Migration/Motility for each time-point.

### Example 1

In the following example cell migration was measured and compared on a gradient-on-chip device provided with migration corridors to the results obtained from gradient-on-chip device without migration corridors. The result is presented as migration directness which is the ratio of migration to motility and indicating how direct a cell or a cell population moves. Cell motility is the total distance travelled by the cell over a certain period of time. Migration directness value is between 0 and 1. A lower migration directness value indicates a higher cell motility deviation from cell migration and therefore a less direct movement.

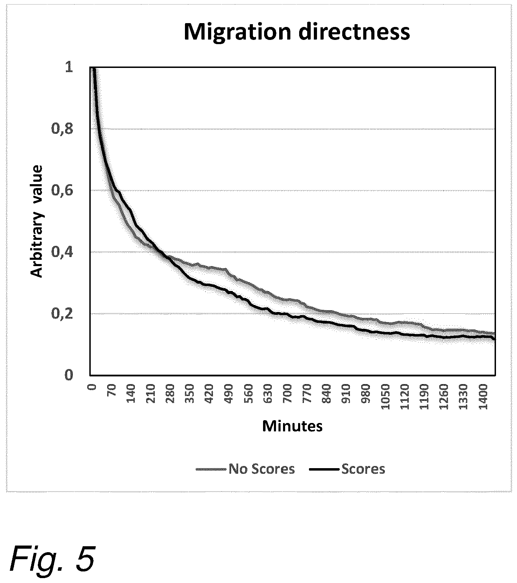

Fig. 5 illustrates that cells initially show a higher migration directness on gradient layers with migration corridors while a lower migration directness after 4 hours compared to the gradient layers with no migration corridors. The migration corridors were delineated by scores which were created randomly in the same orientation as the biomolecule gradient direction and resulted in limitation in cell-cell-interactions. However, the scores also limit the nanosurface and eventually result in a higher cell-cell or cell-edge interactions. Therefore, it is essential to design and induce scores (guiding structure) of exact dimensions to minimize cell-cell interaction over the time.

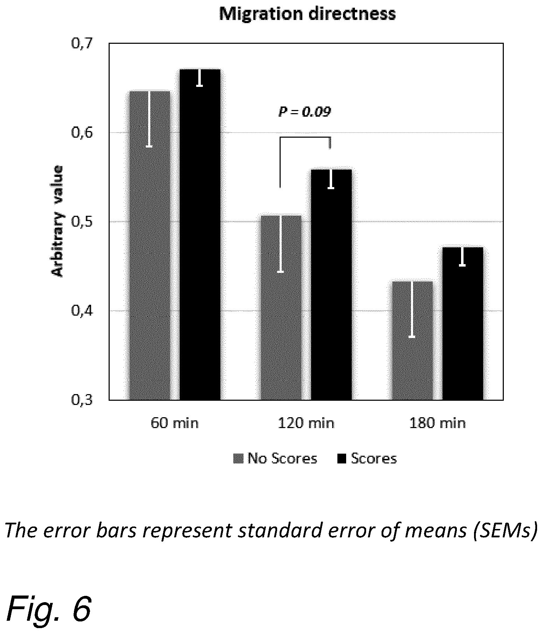

Fig. 6 illustrates a comparison of the cell movement directness 1, 2 and 3 hours after introduction of the cells to the nano gradient layer. Cells move less deviated on the nano gradient layer with scores.

The skilled person realizes that a number of modifications of the embodiments described herein are possible without departing from the scope of the disclosure, which is defined in the appended claims.

## Claims

1. A gradient-on-a-chip device (1) for quantification of cell migration and metastatic potential of tumor cells, said device (1) comprising:
- a chip (10) having a chip surface (11);
- a nano gradient layer of nanoparticles (50) provided on said chip surface (11), said nano gradient layer having a gradient direction (60) along an axis of an X-Y plane of said chip surface (11);
- a biomolecule conjugated to said nanoparticles (50); and
- a linker conjugated to said nanoparticles (50), said linker being adapted to link together said biomolecule to said nanoparticles (50),
**characterized in that** said chip surface (11) having at least one guiding structure (20, 20') arranged to guide said tumor cells in said gradient direction (60), said guiding structure (20, 20') extending in said gradient direction (60) and delineating a migration corridor (30) comprising said nano gradient layer.

2. The device (1) according to claim 1, wherein said guiding structure (20, 20') is a ridge extending out of said chip surface (11) or a recession pointing into said chip surface (11).

3. The device (1) according to any one of claims 1 or 2, wherein said device (1) further comprises a chip surface (11) area void of nano gradient layer, and wherein said guiding structure (20, 20') is the boundary line between said migration corridor (30) and said chip surface (11) area void of nano gradient layer.

4. The device (1) according to any one of claims 1-3, wherein said guiding structure (20, 20') extends continuously along said chip surface (11).

5. The device (1) according to any one of claims 1-4, wherein said migration corridor (30) has a substantially constant width along its extension direction.

6. The device (1) according to any one of claims 1-5, wherein said migration corridor (30) has a width in the range from 20, 20'-500 µm, and preferably from 50-20, 20'0 µm.

7. The device (1) according to any one of claims 1-6, said device (1) further comprising two or more migration corridors (30).

8. The device (1) according to claim 7, wherein said two or more migration corridors (30) are of equal width.

9. The device (1) according to any one of claims 1-8, wherein said linker comprises the linker complex biotin/streptavidin.

10. The device (1) according to any one of claims 1-9, wherein said device (1) further comprises two or more migration corridors (30) provided on top of each other.

11. A method for quantification of cell migration and metastatic potential of tumor cells, said method comprising the steps of:
- applying at least one tumor cell to said gradient-on-a-chip device (1) in claim 1;
- measuring and recording the cell migration of said tumor cell for a repeated time period of 2-48 hours, preferably 3-24 hours, and most preferably 3-12 hours.

12. The method according to claim 11, wherein said measuring and recording is performed by imaging and/or isotope analysis.

13. The method according to any one of claims 11-12, wherein said tumor cells are breast cancer cells, melanoma cancer cells, prostate cancer cells, colorectal cancer cells, or lung cancer cells.

14. Use of the gradient-on-a-chip device (1) in claim 1 for quantification of cell migration and metastatic potential of tumor cells.
